# EUROPEAN PATENT SPECIFICATION

(11) **EP 4 482 496 B1**
(45) Date of publication and mention of the grant of the patent: **03.12.2025**
(21) Application number: 23718963.4
(22) Date of filing: 30.03.2023
(51) Int. Cl.: A61K 31/519, A61P 35/00, A61P 35/04, A61P 37/00

(54) **PAN-CHEMOKINE ANTAGONISTS FOR USE IN TREATING CANCER AND IMMUNE DISORDERS**
PAN CHEMOKINE ANTAGONISTEN ZUR BEHANDLUNG VON KREBS UND IMMUNE KRANKHEITEN
ANTAGONISTES DES PAN-CHEMOKINES POUR TRAITER LE CANCER ET LES MALADIES IMMUNITAIRES

(30) Priority: 04.04.2022 BG 11351722
(43) Date of publication of application: 01.01.2025
(73) Proprietor: "Micar Innovation" Ltd., 1616 Sofia (BG)
(72) Inventor: FRATEV, Filip Filipov, 1407 Sofia (BG)
(74) Representative: Ilieva, Angelina Stanimirova
(86) International application number: PCT/BG2023/000011
(87) International publication number: WO 2023/193070

(56) References cited:
- WO-A2-2004/071460
- DATABASE registry [online] 1 January 2007 (2007-01-01), - ET AL: "920818-76-4 CAS Registry Number: 920818-76-4 REGISTRY Entered STN: 14 Feb 2007 CAS Registry Number Locator: STN Files: CHEMCATS **PROPERTY DATA AVAILABLE IN THE 'PROP' FORMAT** Chemical Name", XP093059615, Database accession no. 920818-76-4
- DATABASE registry [online] 18 April 2023 (2023-04-18), - ET AL: "2920568-49-4 CAS Registry Number: 2920568-49-4 REGISTRY", XP093059617, Database accession no. 2920568-49-4
- DJ SCHOLTEN ET AL: "Pharmacological modulation of chemokine receptor function", BRITISH JOURNAL OF PHARMACOLOGY, WILEY-BLACKWELL, UK, vol. 165, no. 6, 22 February 2012 (2012-02-22), pages 1617 - 1643, XP071170799, ISSN: 0007-1188, DOI: 10.1111/J.1476-5381.2011.01551.X

## Description

### TECHNICAL FIELD OF THE INVENTION

The present invention relates to compounds having chemokine receptors antagonistic activity. One of the compounds is for use in the prevention of metastasis formation in lung, liver and ribs in *in vivo* mice breast cancer model.

### BACKGROUND OF THE INVENTION

CCR7 (C-C chemokine receptor type 7) is a GPCR membrane protein which belongs to the chemokine CCR class. It binds chemokines CCL19 and CCL21, which are important for tissue homeostasis, immune surveillance and tumorigenesis [1]. CCR7 is involved in many diseases such as cancer, where it is critical for the cancer cells to spread by lymph node metastasis, psoriasis and also in arthritis. The overexpression of the CCR7 has mainly been related to lymph node metastasis. It also plays a critical role in the progression of many different malignancies such as breast, gastric, skin (melanoma), head and neck, lung, esophageal, hepatocellular, cervical, thyroid, tonsillar, colorectal and prostate cancers. In most of these cancers, larger tumor size and deeper invasion was associated with CCR7 expression. Chemokines are also very important for the immune response and can be used as a target for treatment of several serious virus infections. For instance, CCR5 is the host receptor by which HIV enter in to human cells and thus is an attractive anti-HIV drug target. The CCR5 antagonist Maraviroc, was approved by the FDA in 2007 in the USA. It showed promising results but unfortunately can cause serious, life-threatening, side effects. Notably, the very recent reports revealed that the C-C chemokines are also significant for the immune response to SARS-CoV-2 virus (Covid-19) and antagonists that simultaneously modulate CCR1, CCR5, CCR7, and CCR8 might be of great help. This was an expected result because earlier it has been also demonstrated the CD4⁺ and CD8⁺ cells importance in Covid-19 infection.

Despite, CCR7 been an attractive drug target a very limited set of molecules which can bind to this chemokine have been reported. A massive highthrouput screen was recently performed and more than 150 000 molecules have been experimentally tested for their CCR7 activity. Fourteen antagonists were detected, mainly small fragments, and also some natural-like molecules including the anti-HIV agent Cosaline by a β-arrestin related assay. The potency of all of these antagonists were about 10µM, except for Cosaline (IC₅₀=0.2µM) [2]. Other type of assays and selectivity studies were not performed. Also, there is a patent US7691856B2 disclosing a class of highly potent antagonists but details about discovery process, structure-activity relationships (SAR) and selectivity etc., has been never published in a scientific report. Another molecule (ICT5888) was used as a starting point in developing of new compound (ICT13069) by University of Bradford but the data is not yet public. However, the recently published PhD thesis revealed the discovery path of ICT13069, which comprise a massive project with SARs and synthesis of over 100 compounds [3]. The activity of this antagonist measured by Ca²⁺ mobilization assay was 0.1µM. Thus, the CCR7 is an attractive target area for both academia and big-pharma.

An X-ray structure of CCR7 in a complex with the most active ligand, cmp2105, of the aftermentioned (US7691856B2) series and binding studies were published by the Roche research group [4]. This research provides valuable structural information and can be used for both the validation of already obtained theoretical models and further examinations. Indeed, it could be employed also for virtual screen campaign and lead optimizations. Documents:
DJ SCHOLTEN ET AL: "Pharmacological modulation of chemokine receptor function", BRITISH JOURNAL OF PHARMACOLOGY, WILEY-BLACKWELL, UK, vol. 165, no. 6, 22 February 2012 (2012-02-22), pages 1617-1643, XP071170799, ISSN: 0007-1188, DOI: 10.1111/ J.1476-5381.2011.01551.X
and WO 2004/071460
disclose chemokine receptors antagonists for use in therapy.

The aim of present invention is to provide compounds having chemokine receptors antagonistic activity i.e. to provide new CC7 antagonist.

### DESCRIPTION OF THE INVENTION

Present invention discloses 2-({6-phenylthieno[2,3-d]pyrimidin-4-yl}oxy)benzamide, denoted also as Hit 1, MIC5022 for use as a new CCR7 active antagonist, which also turned out to be a pan-chemokine antagonist. It discloses further some structure activity relationships (SAR) studies, selectivity studies and several in vitro assays, in an attempt to identify the second messenger path, and also theoretical studies of the binding mode of identified ligand. Invention also discloses the in vivo data of 4T-1-Luc mice breast cancer model which clearly demonstrate the efficacy of 2-({6-phenylthieno[2,3-d]pyrimidin-4-yl}oxy)benzamide (Hit 1, MIC5022) for use in the prevention and reduction of lung, liver and rib metastasis thus showing the treatment potential of this compound.

In another aspect, present invention discloses the chemical formula, synthetic paths and in vitro biological data for two new pan-chemokine antagonist compounds: 3-chloro-2-{[6-(2-chlorophenyl)-2,5-dimethylthieno[2,3-d]pyrimidin-4-yl]oxy}benzamide, denoted also as MIC5023, and 3-chloro-2-{[6-(2-chlorophenyl)-2-methylthieno[2,3-d]pyrimidin-4-yl]oxy}benzamide, denoted also as MIC5024.

### MATERIALS AND METHODS

To facilitate the understanding of this invention, a number of terms are defined below, which have meanings as commonly understood by a person skilled in the art relevant to the present invention.

As used herein, the term "treatment" denotes the use or administration of a therapeutic agent described herein, or identified by a method described herein, to a patient, or use or administration of the therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease, or the predisposition toward disease.

### Virtual screening (VS)

Virtual screen procedures have been already described in inventor's recent studies on AKT1 and GlyT2 inhibitors. Shortly, this procedure combines the structure based pharmacophore and docking screen followed by Induced-fit docking (IFD). For this type of approaches a structure of high quality is also necessary. However, in the current invention, this protocol is further optimized by executing extensive Molecular dynamics (MD) after IFD to obtain a holo-like structures for the best hits and then the compounds were re-docked. It should be underlined that during the research & development and subsequent study activities, no experimental CCR7 structure was available (it was published on PDB in September 2019, PDB code 6QZH) and inventors needed to construct a homology-based model which was refined by many MD simulations and the final output structure was used for the VS camping.

Due to the high flexibility of the GPCRs binding pockets the number of MDs used for obtaining the initial apo structure was increased. For CCR7 inventors used over 10 MD simulations with lengths between 500ns and 1µs. Also, a set of accelerated (aMD) simulations were performed. Based on the obtained data the apo CCR7 structure was retrieved by clustering which was the base for the present structure-based pharmacophore model. The pharmacophore points were obtained after docking of about 600 small fragments into the orthosteric CCR7 binding site. For this model the default setting were not used, which are implemented in the Schrodinger software, but instead 6 pharmacophore points were created and requested all of them to be matched. In addition to the automatically created pharmacophores, all important points, according to both MD and recent biochemical mutations studies, are included and manually added and modified some of these points. The requestment all pharmacophore points to be matched is a highly restrict approach and reduce the number of hits but mating all points have significant benefits and reduced the number of false positive ligands.

Curiously, similar pharmacophore model is valid and can be used also to some extend for the allosteric site. Indeed, the allosteric binding site shapes and dimensions are different than those of the orthosteric one and it is smaller thus only small in size ligands (M.W of about 350 Da) can fit into both binding sites but this is an interesting strategy to find potential cooperative binders. This feature is unique across the CCR family and is present only in CCR7. Invention initial and primary goal was to find orthosteric binders, thus it started the VS screen with this binding site. However, the founded hits then were docked also to allosteric binding site.

Based on orthosteric pharmacophore model over 4 million compounds were screened from ZINC database (lead-like set, version 2017), which were available during initial phase of this study, and also the Enamine hts set (version 2020_2). As a result, only 1300 molecules matched these six key interactions (pharmacophore points) and ligands were further docked and rescored by Schrodinger's Glide SP software. The most promising top 100 hits from each set were visually inspected and then re-docked by the Induced fit docking procedure which takes into account also the flexibility of the binding pocket residues. Next, the output ligand-protein complexes were carefully examined and over 20 ligands were selected for MD and free energy (MM/PBSA) studies. Previously such procedure was not executed but the CCR7 pocket is highly flexible and to find some holo-like structure, e.g. with ligand in the binding pocket, it should be refined by intensive MD simulations.

During the MM/PBSA studies invention also used as a reference the free energy data for recently discovered small molecules and also those from previous studies for which both MD simulations with Charm force field (FF) and experimental data were available. This was necessary because Charm36m FF was used for thr MDs and the AmberTools software for MM/PBSA but this combination was not much, even at all, tested. For instance, if one use Amber14SB FF in a combination with AmberTools will obtain one value from the MM/PBSA calculations which will be significantly different if Charm FF is employed. Thus, to obtain some information about in what free energy range we can expect active ligands all previous data were used as a reference and a value of -20 kcal/mol was set as a threshold for the potentially active hits. As a result it is observed that most of the ligands with a good docking score were not CCR7 binders, according to the MM/PBSA calculations, and some of them even left the binding pocket during the MD simulations which were with length of 300-ns each. These simulations showed the right direction and the most likely holo CCR7 structure has been obtained based on the most active, e.g. with lowest MM/PBSA value ligand and clustering. A new docking was further performed followed by new MD and MM/PBSA studies. Same 1300 molecules were also screened for their capability to bind to the allosteric site. Surprisingly, one of the ligands, named as a Hit 1 here, UPAC name: 2-({6-phenylthieno[2,3-d]pyrimidin-4-yl}oxy)benzamide, (also denoted also as MIC5022), ZINC9862573 was found between the top 10 ranked compounds for both the allosteric and orthosteric sites. Finally, based on all of these computational work and combining the results from the VS camping with apo and holo structures the final set of 16 ligands was selected which to be sent for biological evaluations.

### Biological in vitro data

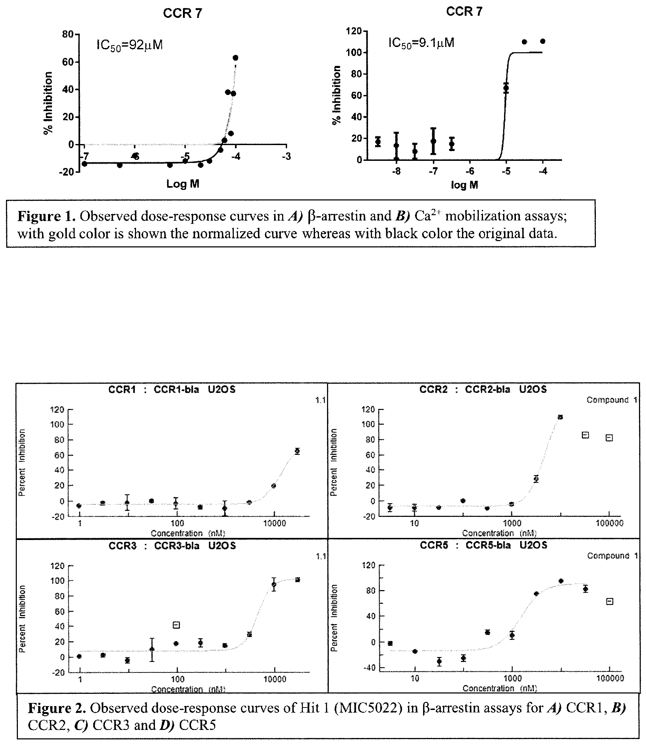

Selected compounds were initially sent for both antagonist and agonist TANGO β-arrestin assays. Among these ligands Hit 1, UPAC name: 2-({6-phenylthieno[2,3-d]pyrimidin-4-yl}oxy)benzamide, (also denoted also as **MIC5022),** ZINC9862573 (also named here as Compound 1, G1 **(Table 1** and **Figure 1A)** showed a promising antagonistic activity, an IC₅₀ value of 9.1µM and no any agonist activity. Further, this compound was tested by Ca²⁺ mobilization assay and a 10 fold difference was obtained (IC₅₀=92µM, **Figure 1B).** This was a strong indication that compound 1 is β-arrestin biased. However, further experiments in an independent lab showed an IC₅₀ in a range of 25-30 µM in similar Ca²⁺ mobilization assay thus the exact bias degree should be taken with caution (data not shown). Because for this small compound (MW=347 Da) even the small changes (methyl, substitutions) may increase significantly its potency it was decided to perform simple structure-activity relationships (SAR) analysis using some commercially available derivatives. Replacement only on the amide group with CN led to strong activity decrease in β-arrestin assay: only 31% activity at about 30µM and similar one at 100µM **(Table 1).** Five more derivatives with replaced amide group were also tested in parallel by Ca²⁺ mobilization assay and they all were not active up to the tested concertation of 100µM.

**Table 1. Structure-activity relationships (SAR) analysis of Hit 1 (MIC5022) derivatives.**

| 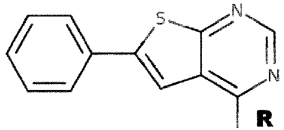 | | | |
|---|---|---|---|
| **Compound** | **R** | **β-arrestin assay** (100µM) *^{a}* | **Ca²⁺ assay** (100µM) *^{b}* |
| 1 Hit 1 | 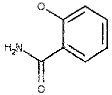 | 110% | 63% |
| 2 | 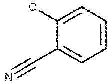 | 30% | N/A |
| 3 | 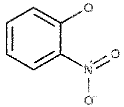 | N/A | < 20 |
| 4 | 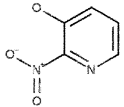 | N/A | < 20 |
| 5 | 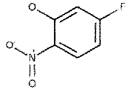 | N/A | < 20 |
| 6 | 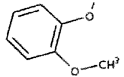 | N/A | < 20 |
| 7 | 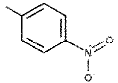 | N/A | < 20 |

| | | | |
|---|---|---|---|
| ^{a, b} Percent of inhibition at 100µM concentration in performed β-arrestin and Ca²⁺ assays; NA- Not available | | | |

Assuming that the difference between these assays (bias) is about 10x this means that these compounds would have activity of no less than 10µM in β-arrestin assay.

Next, series of selectivity assays were performed. Notably, hit 1 was not CCR7 selective but rather pan-CCR chemokine antagonist **(Figures 2A-D).** It was even more active in the β-arrestin assays for CCR2, CCR3 and CCR5 with IC₅₀s of 2.6, 4.4 and 1.9µM, respectively, and less active to CCR1 (IC₅₀=13.6µM). This is an expected result for a compound retrieved as a hit from VS and this results was considered as a positive because Hit 1 can be relative easy transformed, as for example by FEP+ guided lead optimization, to potent and selective compounds related to many therapeutic areas. Moreover, based on the very recent data it seems that such a pan-antagonists can be useful for the Covid-19 infection management and in particular the cytokine storm. Finally, it is not clear whether a pan-antagonist to several known and important for cancer development and metastasis spread chemokines would not provide an additive positive effect in the treatment. Based on these literature data it is believed that development of pan-antagonist to several CCRs would have much more benefits than a selective one.

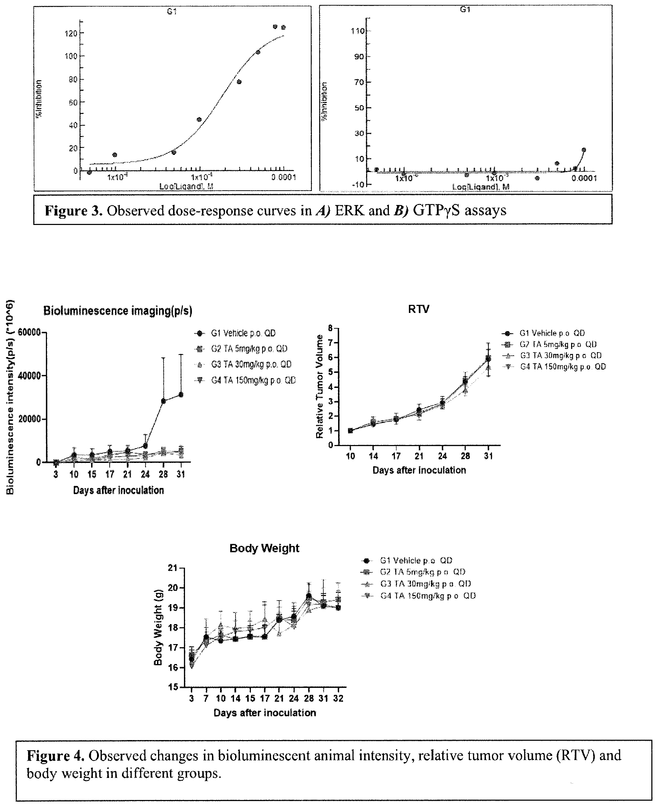

A Ca²⁺ mobilization assay was also performed for other CCRs and an IC₅₀ of 55µM for CCR5 was calculated thus for these chemokine receptors the bias to β-arrestin was even higher. From this assay it became also clear that Hit 1 is a CCR9 antagonist with an IC₅₀ value of 58µM. cAMP assay for CCR5 was also done and a similar value was obtained (IC₅₀=50µM, data not shown). The K_{b} values are also retrieved. For CCR7 and CCR9 K_{b} values of 2.8 and 5.2µM were obtained but for CCR5 it was 10.5µM. Finally, to make it more clear the second messenger output for Hit1 and confirm the nature of β-arrestin bias GTPyS and ERK assays were also performed. Whereas the compound only started to inhibit CCR7 at the highest concentration of 100µM in GTPγS assay (16% antagonist activity) it was clear ERK antagonist with an IC₅₀=18.1µM. Considering that the ERK is directly related to β-arrestin path and binds directly to the arrestin it was concluded that this compound is β-arrestin biased **(Table 1** and **Figures 3A and 3B).** Finally, several other potential hits were identified but the activities were in a range of 25-35% at 100µM concentration and they were discarded in this disclosure.

### An in vitro Proof of concept based on common inflammation processes

Further an *in vitro* prove of concept (PoC) of the observed chemokine activity of Hit 1 was wanted. For this purpose a model for the inhibition of CCL2 release from IL-4, IL-13, IL-22 and IFNy-stimulated primary human keratinocytes (Eczematous inflammation) was used and also an assay for the inhibition of IL-8 release from IL-17A and TNFα (Psoriatic inflammation). These models are similar to the SARS-CoV-2 inflammations assays panels, which become available later and also represent a good PoC for the metastatic processes. As a result Hit 1 acts as an inhibitor of the inflammation and has an effect on CCL2, IL8 and IL17 release. The measured effective concentrations EC₅₀ values were 411nM, 733nM and 3780nM, respectively. Thus, it also seems that Hit 1 has an additive mechanism of action based on its pan-antagonistic nature. This is not an unexpected result because as it was shown above the Hit 1 is a CCR pan-antagonist and in particular modulator of CCR2 and most likely CCR4 activity, which indeed will antagonize the CCL2 release. Many other chemokines are also involved in aftermentioned inflammation processes. Some inhibition of the cell viability was also observed, however, inhibition of cytokine release is often accompanied by some reduction of cell viability, particularly if the molecule is of potency with an EC₅₀ around the micro-molar range. Finally, migration assay by MB231cell line was performed. The addition of 100nM of CCL19/CCL21 increased migratory cells percent from 100% (control) to 200% but Hit 1 abolished this effect by 10 folds (only 20% migratory cells were observed).

### An in vivo proof of concept based on breast cancer 4T-1-Luc⁺ mice model

**Design of the *in vivo* study:** A total of 44 female BALB/c mice (12 mice for maximal tolerance dose (MTD) study; 32 mice for efficacy study) were used in the 4T-1-Luc study. The animals were specific pathogen free and approximately 4-5 weeks old at the initiation of the study.

Murine breast carcinoma cells 4T-1-luc cells used in this study, were cultured in a 37 °C incubator containing 5% CO₂ with 10% FBS and 2µg/mL puromycin in RPMI1640 medium. The cells were sub-cultured within 10 passages before inoculated into the mice. 2*10⁵ 4T-1-luc cells suspended in 20uL PBS were orthotopically injected into the 4^{th} fat pat on the right side of each mouse. Before inoculation, mice were pre-anesthetized with 30mg/kg Zoletil mixed with 10mg/kg Xylazine and kept anesthetized with 3-4% isoflurane. Injected subcutaneously 20mg/kg Tolfedine as painkillers once a day for 3 days. The day of cell inoculation was denoted as Day0. On Day3, 16 bearing-tumor mice were randomized into 4 groups (4 mice per group) according to body weight and BLI signal.

The information of the preparation of Hit 1 dosing solution is as follow:
For 2-({6-phenylthieno[2,3-d]pyrimidin-4-yl}oxy)benzamide (Hit1, MIC5022) 5 mg/kg dose, the preparation was like that: weighed 2.5 mg of Hit 1, added in 0.50ml of ethanol, vortex or magnetic stirring enough to mix well (all compound dissolved will be better), added in 2.00ml of PEG400, vortex or magnetic stirring until getting a clear solution (no more than 60°C water base ultrasonicate may be needed), added 2.50ml of water to get clear solution. The concentration was 0.5 mg/ml, the storage condition was at 4C temperature and the preparation frequency was each 5 days.

For 2-({6-phenylthieno[2,3-d]pyrimidin-4-yl}oxy)benzamide (Hit1, MIC5022) 30 mg/kg dose, the preparation was like that: weighed 15 mg of Hit 1, added in 0.50ml of ethanol, vortex or magnetic stirring enough to mix well (all compound dissolved will be better), added in 2.00ml of PEG400, vortex or magnetic stirring until getting a clear solution (no more than 60°C water base ultrasonicate may be needed), added 2.50ml of water to get clear solution. The concentration was 3 mg/ml, the storage condition was at 4C temperature, and the preparation frequency was each 5 days.

For 2-({6-phenylthieno[2,3-d]pyrimidin-4-yl}oxy)benzamide (Hit1, MIC5022) 150 mg/kg dose, the preparation was like that: weighed 75 mg of Hit 1, added in 0.50ml of ethanol, vortex or magnetic stirring enough to mix well (all compound dissolved will be better), added in 2.00ml of PEG400, vortex or magnetic stirring until getting a clear solution (no more than 60°C water base ultrasonicate may be needed), added 2.50ml of water to get clear solution. The concentration was 15 mg/ml, the storage condition was at 4C temperature, and the preparation frequency was each 5 days.

Each mouse has been observed for changes in general appearance and behavior every day for 4 weeks after grouping. The abnormal phenomena were recorded.

Body weight: Mice were measured and recorded twice a week after grouping, and during the dose period. Animals were also weighed in the event of accidental death or near-death euthanasia. Tumor volume: The tumor volume (V) was calculated as follows: V = (length xwidth2) / 2. Tumor volume was measured twice per week. Tumor growth inhibition (TGI), TGI= (1-(T/C)×100%; T and C as the mean tumor volumes of the treatment and control groups on the measurement day.

The individual relative tumor volume (RTV) was calculated as follows: RTV=Vt/V0, where Vt was the volume on each day, and V0 was the volume at the beginning of the treatment. IVIS imaging: We used IVIS^{®} In Vivo Imaging Systems (PerkinElmer, Waltham, MA) which allow high sensitivity and high resolution in vivo BLI and fluorescence imaging (FLI) across a wide range of wavelengths. Animals were imaged up to five at a time under 3-4% isoflurane gas anesthesia. Each mouse has been injected with D-Luciferin and imaged 10-15 minutes after the injection. BLI signal was quantified in regions of interest (ROIs) drawn around tumors or tissues ex vivo and the signal is expressed as photons per second, representing the flux radiating omni-directionally from the user-defined region once per week post grouping (Day3, Day10, Day14, Day17, Day21, Day24, Day28 and Day31).

### Results of the in vivo study:

**MTD studies:** Initially, 10 BALB/c mice were used for the maximal tolerated dose (MTD) response study determination. The mice were randomized based on body weight and were treated with Hit 1 compound with daily (QD) oral doses of 30mg/kg and 150mg/kg. Body weight was measured daily. The animals did not show abnormal behavior during the experiment and any serious side effects were not observed. A significant (>20%) body lost was not observed.

**Animal body weight and state:** In the cancer study experiment, the body weight of G2, G3 and G4 has no significant difference with the body weight of G1 (p>0.05) **(****Figure 4****).** In addition, there was no significant animal weight loss during the experiment (p>0.05), and animal weight show an overall increase in the experiment. Animals did not show abnormal behavior during the experiment.

**Animal tumor volume (TV), relative tumor volume (RTV) and tumor weight (TW):** The tumor volume (TV) of animals has progressively increased during the experiment. There were no significant differences in TV in G2, G3 and G4 compared with G1 (p>0.05). The TV of G1 on Day10 after inoculation was 90.70 ± 5.57 mm³, and on Day31 after inoculation was 515.73 ± 72.98 mm³. The tumor weight (TW) of G1 was 0.97 ± 0.12 g. The TV result of G1 shows that 4T-1-luc orthotopic model was established successfully in BALB/c mice. The TV of G2 Hit 1 5mg/kg dose group animals on Day10 after inoculation was 86.53 ± 6.03 mm³, and on Day31 after inoculation was 515.90 ± 53.18 mm³. The relative tumor volume (RTV) of G2 on Day31 after inoculation was 5.99 ± 0.61 mm³. The TW of G2 was 0.76 ± 0.23 g. The TV of G3 TA 30mg/kg animals on Day10 after inoculation was 90.30 ± 7.67 mm³, and on Day31 after inoculation was 489.69 ± 85.03 mm³. The RTV of G3 on Day31 after inoculation was 5.33 ± 0.64 mm³. The TW of G3 was 0.95 ± 0.16 g. The TV of G4 TA 150mg/kg animals on Day10 after inoculation was 85.22 ± 10.26 mm³, and on Day31 after inoculation was 515.66 ± 98.37 mm³. The RTV of G4 on Day31 after inoculation was 5.92 ± 0.63 mm³. The TW of G4 was 1.05 ± 0.19 g. The TV, RTV and TW of G2, G3 and G4 has no significant differences compared with G1 in the experiment (p>0.05) *but in G3 group we observed a clear decrease in the RTV after day 24* **(****Figure 4****).** Thus, based on these data we can conclude that Hit1 induce a similar reduction in the tumor size and can be combined with some known drugs for further achievement of the tumors management.

**Animal bioluminescence imaging and metastasis on organs:** In the experiment, animals have been processed bioluminescence imaging on Day3, Day10, Day15, Day17, Day21, Day28 and Day31 after inoculation to detect the growth of tumor cells. The bioluminescence intensity of G1 has sustained increased **(****Figures 4** (top left) and **5A).** The bioluminescence intensity of the control group (G1) on Day3 after inoculation was 0.23*10⁸± 0.081*10⁸ p/s, and on Day31 after inoculation was **31.31*10⁸ ± 18.39*10⁸ p/s (Table 2).**

The bioluminescence intensity result of G1 shows that 4T-1-luc Orthotopic model was established successfully in BALB/c mice.

The bioluminescence intensity of treatment G2 (5 mg/kg) on Day3 after inoculation was 0.21*10⁷ ± 0.056*10⁸ p/s, and on Day31 after inoculation was **5.01 *10⁸** ± **2.67*10⁸ p/s.**

The bioluminescence intensity of G3 group (30 mg/kg) on Day3 after inoculation was 0.23* 10⁷ ± 0.075*10⁸ p/s, and on Day31 after inoculation was **3.26*10⁸** ± **0.89*10⁸ p/s.**

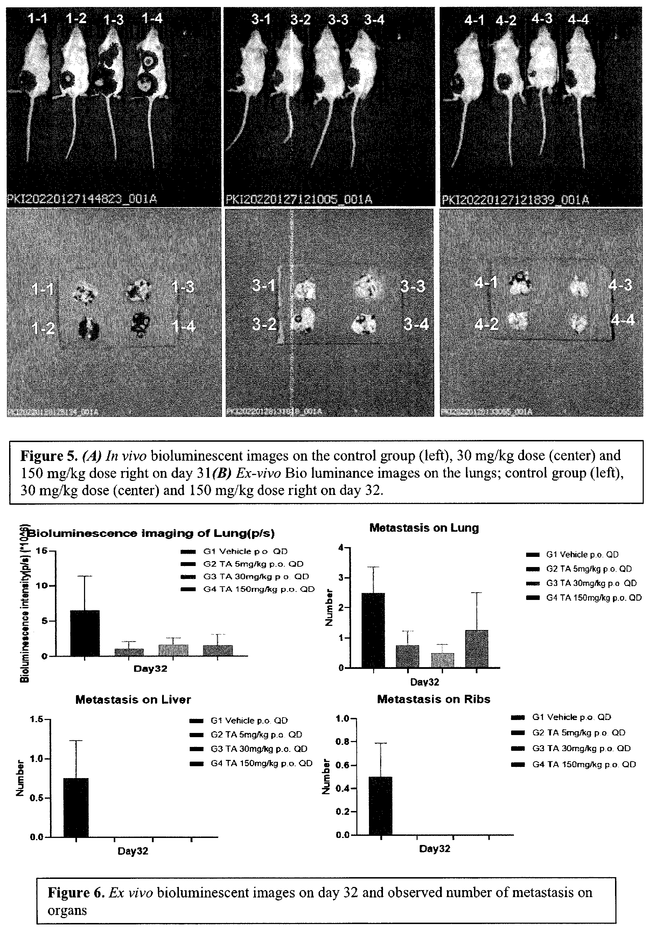

The bioluminescence intensity of G4 group (150 mg/kg) on Day3 after inoculation was 0.21*10⁷ ± 0.054*10⁸ p/s, and on Day31 after inoculation was **5.08*10⁸** ± **2.16*10⁸ p/s. These results showed that *the spread of the tumor has been greatly reduced by 6-10 folds.*** For the result of bioluminescence imaging of lung **(Figures 5B and** **6****)** that processed on Day32 after inoculation, the bioluminescence intensity of the control G1 group was the highest **(6.48*10⁶** ± **4.91*10⁶),** whereas for the 5, 30 and 150mg/kg treatment groups only **1.07E+06, 1.66E+06** and **1.56E+06,** respectively, ***i.e. the metastasis development was reduced about five times.*** Based on the *ex-vivo* studies, the metastasis on organs of the control G1 group **(Table 3)** happened most in lung, and all detected organs of G1 (lung, liver and ribs) have found metastasis. The metastasis on lung is 3/4, the metastasis on liver is 2/4 and the metastasis on ribs is 2/4. The metastasis on organs of G2, G3 and G4 happened only in lung.

The metastasis on lung for the treatment groups G2 and G3 is 2/4, and for G4 is 1/4, respectively **(****Figure 6** and **Table 3).** *The total number of metastasis found by the ex-vivo studies in the control group was 10, whereas in the treatment groups with 5, 30 and 150 mg*/*kg doses of Hit 1 compound were only 3, 2 and 5, respectively.*

**Based on these data one can conclude that a notable reduction in both metastasis and tumor spread have been observed. The spread of the tumors was reduced by folds according to *in vivo* bioluminescence images (****Figure 4****) and also greatly reduced in lungs as it is evident from the *ex-vivo* bioluminescence images (****Figure 6****). The metastasis in liver and ribs have been completely eliminated. This was clearly visible on all images. The *ex-vivo* manual counting confirmed that.**

**Conclusions from the *in vivo* study:** the 4T-1-luc Orthotopic model was established successfully in BALB/c mice, and the tumor volume (TV) of G1 has continued increase during the experiment. The TV on Day31 after inoculation was 515.73 ± 72.98 mm³. During the experiment, there was no mice death or abnormal behavior of mice. In addition, compared to the beginning of the experiment,

**Table 2. Animal bioluminescence imaging intensity (10⁸ p/s)**

| **Day** | **G1 Vehicle p.o. QD** | | **G2 Hit1 5mg/kg p.o. QD** | | **G3 Hit1 30mg/kg p.o. QD** | | **Q4 Hit1 150mg/kg p.o. QD** | |
|---|---|---|---|---|---|---|---|---|
| | **Mean** | **SEM** | **Mean** | **SEM** | **Mean** | **SEM** | **Mean** | **SEM** |
| 3 | 2.28E+07 | 8.12E+06 | 2.07E+07 | 5.57E+06 | 2.31E+07 | 7.53E+06 | 2.07E+07 | 5.39E+06 |
| 10 | 3.54E+08 | 3.21E+08 | 1.56E+08 | 8.29E+07 | 2.71E+08 | 7.62E+07 | 4.97E+07 | 1.06E+07 |
| 15 | 3.44E+08 | 2.83E+08 | 1.69E+08 | 6.11E+07 | 1.09E+08 | 3.10E+07 | 1.33E+08 | 7.42E+07 |
| 17 | 4.94E+08 | 3.00E+08 | 3.39E+08 | 1.10E+08 | 1.32E+08 | 3.70E+07 | 2.43E+08 | 1.13E+08 |
| 21 | 5.35E+08 | 2.44E+08 | 4.78E+08 | 2.35E+08 | 1.30E+08 | 1.59E+07 | 2.94E+08 | 1.54E+08 |
| 24 | **7.62E+08** | 5.19E+08 | **3.52E+08** | 1.07E+08 | **2.08E+08** | 5.07E+07 | **3.20E+08** | 9.58E+07 |
| 28 | 2.84E+09 | 1.98E+09 | 4.34E+08 | 1.28E+08 | 4.49E+08 | 2.19E+08 | 5.27E+08 | 1.77E+08 |
| 31 | **3.13E+09** | 1.84E+09 | **5.01E+08** | 2.67E+08 | **3.26E+08** | 8.94E+07 | **5.08E+08** | 2.16E+08 |

**Table 3. Metastasis on organs**

| **Metastasis on organs** | **Metastasis on Lung** | **Metastasis on Liver** | **Metastasis on ribs** |
|---|---|---|---|
| **G1** (control) | (3/4 mice) totally 10 | (2/4 mice) totally 3 | (2/4 mice) totally 2 |
| **G2** (5mg/kg) | (2/4 mice) totally 3 | (0/4 mice) totally 0 | (0/4 mice) totally 0 |
| **G3** (30mg/kg) | (2/4 mice) totally 2 | (0/4 mice) totally 0 | (0/4 mice) totally 0 |
| **G4** (150mg/kg) | (1/4 mice) totally 5 | (0/4 mice) totally 0 | (0/4 mice) totally 0 |

the body weight of mice has increased at the end. It can be considered that the Hit 1 is relatively biologically safe. The TV, RTV, TW and the results of bioluminescence imaging of 4^{th} fat pat and lung of G2 Hit 1 5mg/kg have continual increase and have no significant differences compared with G1 in the experiment. It can be considered that Hit 1 with 5mg/kg concentration has less effect on inhibiting the growth of tumor. However, the result of metastasis on organs shows test article is effective in inhibiting metastasis especially metastasis on liver and ribs. Similarly, the TV, RTV, TW and the results of bioluminescence imaging of 4^{th} fat pat and lung of G3 Hit 1 30mg/kg have continual increase and have no significant differences compared with G1 in the experiment. Therefore, it can be considered that test article with 30mg/kg concentration has less effect on inhibiting the growth of tumor. However, the result of metastasis on organs shows Hit 1 with 30mg/kg concentration is effective in inhibiting metastasis especially metastasis on liver and ribs as well.

The TV, RTV, TW and the results of bioluminescence imaging of 4^{th} fat pat and lung of G4 Hit 1 150mg/kg have continual increase and have no significant differences compared with G1 in the experiment. It can be considered that test article with 150mg/kg concentration has less effect on inhibiting the growth of tumor. The result of metastasis on organs shows test article with 150mg/kg concentration is relatively effective in inhibiting metastasis especially metastasis on liver and ribs, and the inhibition was better than 30mg/kg concentration and 5mg/kg concentration.

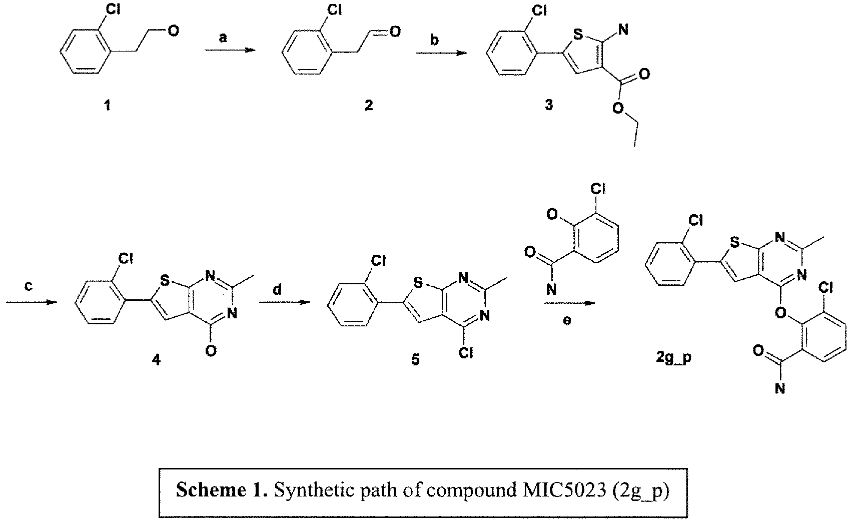

### MIC5023 (IUPAC name: 3-chloro-2-{[6-(2-chlorophenyl)-2,5-dimethylthieno[2,3-d]pyrimidin-4-yl]oxy}benzamide) and MIC5024 (IUPAC name: 3-chloro-2-{[6-(2-chlorophenyl)-2-methylthieno[2,3-d]pyrimidin-4-yl]oxy}benzamide) are new pan-chemokine antagonists

In another aspect of the invention after identification of the binding sites (see below) and theoretical Free energy of perturbations calculations (FEP) new pan-chemokine antagonists were generated, which were further chemically synthesized and biologically evaluated. The aim of the invention was to further identify and confirm the binding mode of the compounds, and also some ADMET profiles, thus, this set can be considered as a preliminary set of compounds which can be used in the further lead optimization process. Between these analogues the compounds 3-chloro-2-{[6-(2-chlorophenyl)-2,5-dimethylthieno[2,3-d]pyrimidin-4-yl]oxy}benzamide, denoted also as **MIC5023,** and 3-chloro-2-{[6-(2-chlorophenyl)-2-methylthieno[2,3-d]pyrimidin-4-yl]oxy}benzamide, denoted also as **MIC5024,** were synthesized and sent for biological evaluations.

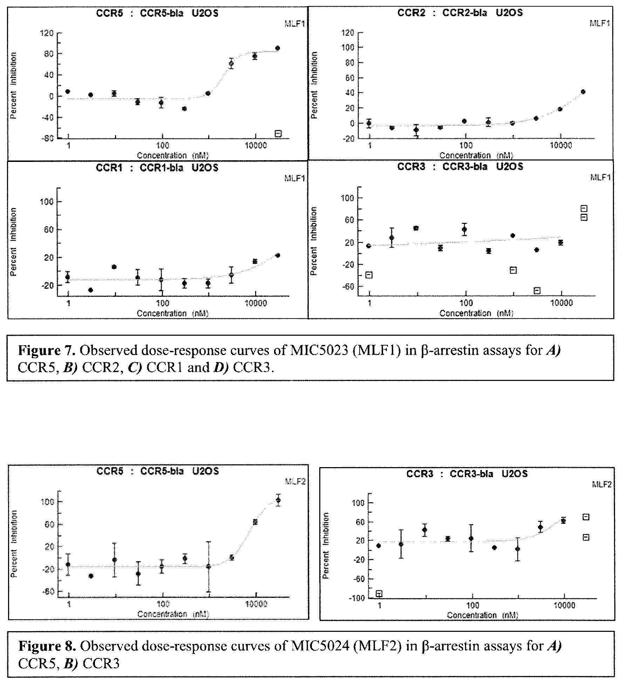

**MIC5023.** The synthesis path of MIC5023 is shown on **Scheme 1.** It consists of 5 chemical transformation in order the final product to be obtained. Two chlorine atoms were added to aromatic rings of MIC5022, and also one methyl group, which makes MIC5023 a close derivative of MIC5022. As a result the MIC5023 showed an activity to several chemokines. The CCR5 β-arrestin activity was similar to that of MIC5022 and IC₅₀ of 2.1µM was obtained **(****Figure 7****).** However, the activity to CCR2 was greatly reduced to IC₅₀ of 31.5 µM. The activity to CCR1 was in particular abolished (IC₅₀ >30µM) but for CCR3 was only slightly reduced (IC₅₀ >9.5µM). The Ca²⁺ mobilization assays retrieved a value of IC₅₀ of about 30µM and similar for CXCR4; i.e. less than those observed for Hit 1 (MIC5022).

**MIC5024.** The synthesis path of MIC5024 is shown on **Scheme 2.** It consists of 4 chemical transformation in order the final product to be obtained. The difference between MIC5023 and MIC5024 is only one methyl group introduced in the central five member aromatic ring but it decreased the β-arrestin activity of MIC5024 to CCR1, CCR2 and CCR3 below 30µM. The activity to CCR5 was also reduced to an IC₅₀ value of 7.2µM **(****Figure 8****).** However, the activity to CCR3 (IC₅₀ =7.2µM) was similar to Hit 1 (MIC5022) and MIC5023. The Ca²⁺ mobilization assays retrieved values of IC₅₀>50µM for CCR7 and CXCR4. Thus, it became evident that this methyl substitution is less sensitive only to CCR3 and CCR5. This clearly revealed both the potential biding mode of these compounds and an idea of the further optimization steps.

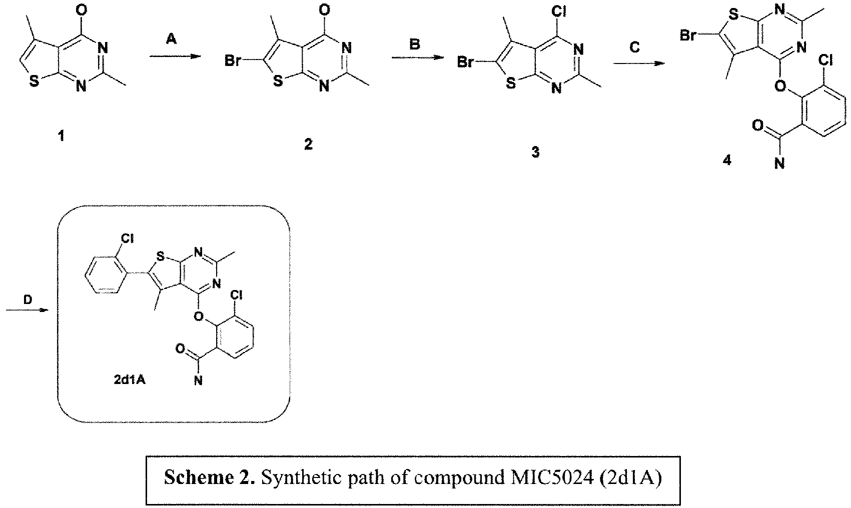

### Counterscreen and an analysis of the observed Hill coefficients

The CCR7 dose-response curve **(Table 1, Figures 1A and 1B)** was very steam, which is usually a "red flag" for an artefact. One common indication of artifact among hits is a steep dose-response curve, often indicated by a high Hill coefficient. For such compounds, inhibition rises much more quickly with concentration than one would expect. Three mechanisms may be considered for high Hill coefficients. First, several molecules may bind to one receptor; here, the slope of the dose-response curve will rise with number of inhibitor sites. Second, the inhibitor may undergo a physical phase transition, such as colloid aggregation, as its concentration is raised. Colloid formation have a sharp concentration dependence, and if this transition is coupled to inhibition, a steep dose-response curve will result. Finally, steep dose-response curves will occur in any enzyme-inhibitor pair when the enzyme concentration significantly exceeds the *K*_{d} value of the inhibitor, which is obviously not the case in this study.

In current CCR7-β-arrestin antagonistic assay a Hill coefficient of 15.1 was obtained, without any transformation of the data. Instead for CCR1, CCR2, CCR3 and CCR5 coefficients much closer to one were calculated, 2.3, 3.1, 2.6 and 1.9, respectively. Moreover, the increase of the activity for CCR3 and CCR5 were over 50-folds for concentrations where the activity raised. The calculations for CCR7 using the same data but transformed to log units, normalized and employing GraphPad 6 software yielded an IC₅₀=9.2µM and Hill slope of 11.2 **(Figure 1A).** Further, the CCR7-β-arrestin antagonistic assay was repeated and retrieved almost identical results. From Ca²⁺ mobilization assay Hill Slope of 2.6 for the CCR7 data was obtained which indicates that observed high value of the Hill coefficient is mainly related to β-arrestin antagonistic assay.

To examine the colloid aggregation possibility a β-lactamase enzymatic activity counterscreen assay was performed, which is the commonly adopted tool for detection of such properties of the compounds. The results clearly showed no activity on β-lactamase (-18% at 50µM concertation). Also, the preliminary data with 0.01 % Triton X-100 reagent, which is known to disturb the colloid formations, added into the Ca²⁺ mobilization assay additionally shown no activity change in CCR7 expressed cells confirming that the aggregation is not the reason and an appropriate explanation for the observed steep dose-response curves and Hill coefficient as well as.

Current diversity data provide also several additional experimental evidences which are against colloid aggregation activity dependence:
- Almost identical steep dose-response curves have been observed for the natural CCL19 and CCL21 agonists. Thus, it seems that this is not unique to Hit 1 case.
- Hill coefficient of 1.5 in the ERK experiments and no steep slope **(Figure 3A).** Moreover, the ERK dose-response curve looks like a typical single antagonist ligand.
- At the same conditions as in the ERK assay the compound was in particular not active in the GTPγS assay up to 100µM **(Figure 3B).** Considering that the colloid inhibition is present at concentrations similar to the aggregate point this is a strong argument against such type of Hit 1 binding.
- The same as above is valid for Ca²⁺ assays on CCR5 and CCR9 and the Hill-Slopes were small 1.2 and 0.8, respectively. Moreover, the observed Ca²⁺ activity is much lower and it would be difficult an aggregation concentration point to be present in a so wide range; e.g. in a rage of 1 to 100µM. For instance, it is well known that the critical aggregation concentration (CAC, or CMC) for aggregators is compound-specific (e.g., some compounds may aggregate at 5µM, whereas others may only aggregate at 50µM compound concentrations). It is also well known that after reaching the CAC, the surface tension remains relatively constant.
- SARs studies did not show same behavior for very similar compounds that have even higher LogP, which is one of the main predictors for aggregation.

Based on the abovementioned arguments one can conclude that the observed in the β-arrestin assay activity of Hit 1 to CCR7 is not due to colloid aggregation and relates to other mechanisms. The Hill coefficients are usually also considered as a proof for multiple ligand binding. The large variations of reported Hill coefficients most likely corresponds to multiple binding. The commonly adopted hypothesis for such observations is that certain ligands may promote positive binding cooperativity. Thus, experiments further concentrated to explain observed experimental data based on the multiple binding mode mechanism. Such mechanism of action is not unique across the chemokine family. For instance, the crystal structure of CCR2 has shown that it is possible two different ligands to bind in the same time into two completely different binding sites - one in the allosteric and one in the orthosteric site. The structure suggests an interesting symmetrical mechanism for the concurrent antagonistic action of the two compounds. BMS-681 interferes with chemokine binding directly and with G-protein coupling indirectly, by stabilizing an inactive, presumably G-protein-incompatible, conformation of the receptor. Conversely, CCR2-RA-[R] directly prevents G-protein/β-arrestin coupling and allosterically inhibits binding of the CCL2 chemokine, which, like most GPCR agonists, requires an active, G-protein-associated receptor for high affinity binding. Bi-directional allosteric communication between the extra- and intracellular sides of the receptor is reminiscent of that previously observed in adenosine A2A receptor (AA2AR) and β2AR using allosteric inverse agonist antibodies/nanobodies that target the same epitope as CCR2-RA-[R]. Similar to these antibodies, CCR2-RA-[R] was previously shown to allosterically enhance, and to be allosterically enhanced by, binding of orthosteric antagonists, demonstrating positive binding cooperativity.

### Unique binding mode of Hit 1 in CCR7

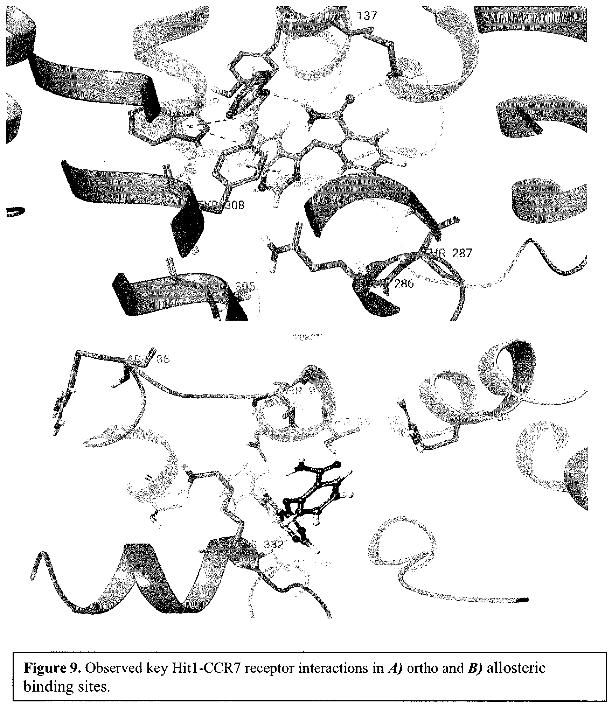

Based on the aftermentioned experimental data for of Hit 1 and also the similar docking scores for the orthosteric and allosteric sites in CCR7 invention decided to elucidate the binding mechanism of the compound in this chemokine. This is also an initial and important step for the further FEP+ guided lead optimization process. Multiple regular MD simulations and Metadynamics in a case when the ligand is present in either the ortho or allosteric sites were run and the ligand binding when both of these sites were simultaneously occupied by the compound was studied. Initially, the receptor residues which interact with Hit 1 in the ortho and allosteric sites were identified based on previous MD holo structures obtained during the VS. This gave an insight into both the binding mode of the ligand and also the key receptor residues which are significant for the binding. For instance, the Lys137, Tyr136, Tyr279 and Tyr308 (tyrosine tirade) were determining for the binding in the ortho site providing H-bonds to the amide group and π-π stacking with aromatic parts of the compound. Most of these residues are unique across the CCR family **(Figure 9A).** Also, the MD simulations showed that the conserved Trp114, which is not significant for selectivity, is also important for the stable ligand binding. In the allosteric site the compound forms H-Bonds with more conserved residues Thr93 and Asp94 and also the Lys332 **(Figure 9B).** The aromatic skeleton was stabilized mainly by Va179, Leu98, Phe333 and Tyr326.

Further, the compound was docked to the recently obtained CCR7 X-ray structure (PDB: 6QZH). Hit 1 fits well to the allosteric site and had identical ligand-receptor interactions but the orthosteric site was closed in this structure, opposite to the MD refined homology model which was used for the VS. Thus, for the ortho site 2x800ns MD simulations were run and in both of them the ligand slowly moved to and stabilized in the predicted during the VS binding conformation. During this path Hit1 was initially stabilized mainly by Gln286 and Asn306 and then moved to the already identified ortho site where the interactions with Lys137, Tyr136, Tyr279, Tyr308, Gln286 and Asn305 were predominated.

The main differences in the observed interactions in the aforementioned holo structure were the percentage of H-bonds formed with individual members of tyrosine tirade and inclusion of Gln286 in a formation stable hydrogen bond. Also, the amide group was not stable and the amino group changed its orientation during the simulations into direction toward both the extra and intracellular space; e.g. about 20-30% of the simulation times it was stabilized by Gln286 and Asn306 not by the tyrosine tirade. A ligand free energy of binding of -24 kcal/mol was calculated by simple MM-PBSA approach. This data indicate that the Hit 1 can bind to the ortho CCR7 binding site.

The same procedure was repeated for the allosteric site and 2x500ns simulations were executed. The ligand moved in parallel to the helices 3, 6 and 7 and the amide group was with an intercellular direction. Significant deviation in the RMSD of the ligand was observed in all of the executed MDs. Initially it was stabilized by the Thr93, Asp94 and Lys332 but the amide group was very flexible. Moreover, the Asp92 and Lys332 can form also a stable hydrogen bond and the ligand was able to form H-bonds with all of the aftermentioned residues. Also, the ligand was also able to form hydrogen bond with Arg114 and interacts with several other residues. The free energy of binding was higher and equal to -21 kcal/mol, according to MM/PBSA calculations.

Further 2x500ns MD simulations were also executed, named as a MD3 and MD4 here, with a ligand present in both the ortho and allosteric binding sites. Also, a 300ns-long Metadynamics calculations using the RMSD changes of the Hit1 in both sites as a collective variable were also performed. The MD3 simulation shown that in a presence of ligand in allosteric binding pocket, the compound in the ortho one was much more stabilized than in a case when only a single ligand is bound to the ortho pocket. During the simulation MD4 very fast unbinding of the ligand in allosteric site was observed after about 80ns. Initially, the amide group of the ligand rotated and interacted with Arg144 disrupting the important for the communication with the ortho site Arg144-helix 6 residue contacts, as it was shown by the simulations of the apo receptor (data not shown).

Further, Hit 1 translated above Arg144 and then totally unbounded into intracellular space. The compound in the ortho site changed it conformation too. The amide group rotated and formed H-bond with ELC2 residues Ser211 and Tyr308 thus bridging the loop with helix 7. Fuhrer, the ligand in the ortho site changed it conformation more and interacted mainly with ECL2 and helix6/7 residues which actually led to the total unbinding of the compound in the allosteric site. The simple RMSD analysis shown that the confrontational changes of the ligand in the allosteric pocket directly correlated with those in the ortho one. Thus, this is a strong indication that the processes in these sites are linked.

The results from regular MD4 run were also supported by well-tempered Metadynamics simulation although the amide group was more unstable in the ortho site and the ligand in the allosteric pocket showed two main conformations. The free energy difference between these conformations was only about 1 kcal/mol which means that they are all likely. It became also evident that when two ligands are bond to the CCR7 sites then the compound in the ortho site can move to different positions which were detected by executed regular MDs and somehow described the binding path already described by these simulations. Also, this metadynamics simulation was similar to MD4 because the amide group of the ligand in allosteric site was close and interacted with Arg154. In turn the ligand in the ortho site was more unstable. The free energy difference between the amide group conformation toward the Gln286 and Asn305 and tyrosine tirade was only 0.2 kcal/mol. Six independent metadynamics simulations were also executed using only RMSD of the ligand in the allosteric pocket as a collective variable and results were similar. The compound always changed it conformation by RMSD of about 1.5Å-2.5Å with a second energy minima's which were closer to its initial position.

Based on all these data and those from the experimental studies one can conclude that in CCR7 Hit 1 has a unique binding mechanism. Invention propose that a cooperative binding seems to be responsible for observed steam dose-response curves and that the ligand can bind to both the ortho and allosteric sites indeed with different probability and frequency. The compound can initially bind to the ortho site and after reaching some concentration, also for a shorter time to the allosteric pocket stabilizing the ligand in the ortho one. After that Hit 1 leave the allosteric site thus the ligand in this pocket acts as allosteric modulator. It is also possible that a second ligand can be also adopted to the ortho site after the compound leave the allosteric pocket. This might be result of the conformational changes in the orthosteric binding site due to the ligand binding in allosteric pocket, which seems to be significant. Such cooperative binding is not rare in the GPCR ortho sites but the allosteric pocket is usually close the ortho one. In the CCR7 case the allosteric site is in a long distance from the ortho pocket and the ligands are approximately separated by 31Å distance. Thus, it seems that Hit 1 has a unique mechanism of action not only between chemokines but also across all known GPCRs. It is also known that CCR7 makes oligomers which additionally increases the number of the total number of compounds bound to the system.

### Binding mode of Hit1 in other chemokines

In another aspect the invention studied also the binding mode of Hit 1 in the other chemokines. Based on both docking and executed MDs it became clear that the compound acts as an allosteric antagonists in these chemokines. The ligand was not able to accommodate into the orthosteric sites simply because the residues involved in the interactions and described in the case of CCR7 above are mutated and cannot provide any reasonable mechanism of binding. On the other hand, the interactions in allosteric sites, where the residues between all CCRs are mostly conserved, were stable and may explain observed activities in CCR1, CCR2, CCR3 and CCR5. For instance, Lys56, Thr64 and Asp66 seems to play significant role in the Hit 1 binding in CCR5. In CCR1 and CCR3 Asp66 is mutated to Ser and Asn, respectively, which can explain decreased activity especially in CCR1. Further, in CCR2 the Ser63 is mutated to Cys which may also influence the ligand-receptor interactions in this part of the binding pocket. However, in CCR7 and CCR9 these residues are identical to those in CCR5, except Lys59 which is mutated to Arg, but a significant decrease in the activity was observed only in CCR7. Finally, identified as a significant for the binding residue Lys332 in CCR7 was mutated to Glu in CCR5 and Arg in CCR1 and CCR9. Thus, these changes indicates for different binding mode of Hit 1 to chemokine family. It is also likely that Glu303 in CCR5 (Lys302 in CCR7) might be involved in the amide group interactions with the receptor in a different way than those in CCR7 leading to the observed increase in activity. In summary, whereas this mutational analysis can at least partly explain observed selectivity in CCR1, 2, 3, 5 and 9, in CCR7 it cannot provide a reasonable explanation of decreased activity compared to CCR5. This means that the discovered ligand has a unique binding properties; it is a pan-chemokine antagonist but acts by different mechanisms in the individual CCR members and in particular CCR7.

The inventive compounds have chemokine receptor antagonistic activity, and thus, are effective for use in the prevention or treatment of diseases associated with the chemokines receptors which are, specifically, lung, liver, ribs and lymph node metastasis and the progression of many different malignancies such as breast, gastric, skin (melanoma), head and neck, lung, esophageal, hepatocellular, cervical, thyroid, tonsillar, colorectal and prostate cancers and the like. Herein, invention discloses MIC5022 for use in the prevention of metastasis formation and development in lung, liver and ribs in *in vivo* mice breast cancer model. MIC5022 has an antagonistic activity to CCR7 mainly due to the presence of amide chemical group in its structure; it affects more the β-arrestin and ERK second massager paths when binds to CCR7 than the Ca2+ and cAMP concentrations levels; e.g. is β-arrestin biased. The compound is also for use in the prevention or treatment of virus infection diseases associated with chemokine receptors such as HIV-1, HIV-2, SARS-CoV-2 (COVID-19) and the like. Also, it is for use in the immune related diseases such as psoriasis, arthritis and atopic dermatitis.

## Claims

1. Compound with IUPAC name: 2-({6-phenylthieno[2,3-d]pyrimidin-4-yl}oxy)benzamide and pharmaceutically acceptable salts thereof for use as a medicament as a CCR7 (C-C chemokine receptor type 7) receptor antagonist.

2. Compound according to claim 1 for use as a pan-chemokine antagonist with an antagonistic effect to CCR1, CCR2, CCR3, CCR5, CCR9 and CXCR4 chemokine receptors.

3. Compound according to claim 1 or 2 for use in the prevention or treatment of cancer metastasis formed in the lung, liver, rib and lymph nodes.

4. Compound according to claim 1 or 2 for use in the reduction of different malignancies' progression, such as breast, gastric, skin (melanoma), head and neck, lung, esophageal, hepatocellular, cervical, thyroid, tonsillar, colorectal and prostate cancer.

5. Compound according to claim 1 or 2 for use in the prevention or treatment of virus infection diseases associated with chemokine receptors such as HIV-1, HIV-2, SARS-CoV-2 (COVID-19).

6. Compound according to claim 1 or 2 for use in the prevention or treatment of immune related diseases such as psoriasis, arthritis and atopic dermatitis.

7. Compound with IUPAC name: 3-chloro-2-{[6-(2-chlorophenyl)-2,5-dimethylthieno[2,3-d]pyrimidin-4-yl]oxy}benzamide (MIC5023) and pharmaceutically acceptable salts thereof.

8. Compound according to claim 7 for use as a pan-chemokine antagonist with an antagonistic effect to CCR1, CCR2, CCR3, CCR5, CCR7 and CXCR4 chemokine receptors.

9. Compound with IUPAC name: 3-chloro-2-{[6-(2-chlorophenyl)-2-methylthieno[2,3-d]pyrimidin-4-yl]oxy}benzamide (MIC5024) and pharmaceutically acceptable salts thereof.

10. Compound according to claim 9 for use as a pan-chemokine antagonist with an antagonistic effect to CCR1, CCR2, CCR3, CCR5, CCR7 and CXCR4 chemokine receptors.

## Patentansprüche

1. Verbindung mit IUPAC-Namen: 2-({6-Phenylthieno[2,3-d]pyrimidin-4-yl}oxy)benzamid und deren pharmazeutisch verträgliche Salze zur Verwendung als Arzneimittel als CCR7 (C-C-Chemokinrezeptor Typ 7) Rezeptorantagonist.

2. Verbindung gemäß Anspruch 1 zur Verwendung als Pan-Chemokin-Antagonist mit antagonistischer Wirkung auf CCR1, CCR2, CCR3, CCR5, CCR9 und CXCR4 Chemokinrezeptoren.

3. Verbindung gemäß Anspruch 1 oder 2 zur Verwendung bei der Prävention oder Behandlung von Krebsmetastasen, die in der Lunge, Leber, Rippe und Lymphknoten gebildet werden.

4. Verbindung gemäß Anspruch 1 oder 2 zur Verwendung bei der Verringerung des Fortschreitens verschiedener maligner Erkrankungen, wie Brust-, Magen-, Haut-(Melanom), Kopf- und Hals-, Lungen-, Ösophagus-, hepatozellulärer, Gebärmutterhals-, Schilddrüsen-, Tonsillen-, kolorektaler und Prostatakrebs.

5. Verbindung gemäß Anspruch 1 oder 2 zur Verwendung bei der Prävention oder Behandlung von Virusinfektionskrankheiten, die mit Chemokinrezeptoren assoziiert sind, wie HIV-1, HIV-2, SARS-CoV-2 (COVID-19).

6. Verbindung gemäß Anspruch 1 oder 2 zur Verwendung bei der Prävention oder Behandlung von Erkrankungen des Immunsystems wie Psoriasis, Arthritis und atopische Dermatitis.

7. Verbindung mit IUPAC-Namen: 3-Chlor-2-{[6-(2-Chlorphenyl)-2,5-dimethylthieno[2,3-d]pyrimidin-4-yl]oxy}benzamid (MIC5023) und deren pharmazeutisch verträgliche Salze.

8. Verbindung gemäß Anspruch 7 zur Verwendung als Pan-Chemokin-Antagonist mit antagonistischer Wirkung auf CCR1, CCR2, CCR3, CCR5, CCR7 und CXCR4 Chemokinrezeptoren.

9. Verbindung mit IUPAC-Namen: 3-Chlor-2-{[6-(2-Chlorphenyl)-2-methylthieno[2,3-d]pyrimidin-4-yl]oxy}benzamid (MIC5024) und deren pharmazeutisch verträgliche Salze.

10. Verbindung gemäß Anspruch 9 zur Verwendung als Pan-Chemokin-Antagonist mit antagonistischer Wirkung auf CCR1, CCR2, CCR3, CCR5, CCR7 und CXCR4 Chemokinrezeptoren.

## Revendications

1. Composé avec nom IUPAC : 2-({6-phénylthiéno[2,3-d]pyrimidin-4-yl}oxy)benzamide et ses sels pharmaceutiquement acceptables pour utilisation comme médicament en tant qu'antagoniste du récepteur CCR7 (récepteur de chimiokine C-C de type 7).

2. Composé selon la revendication 1 pour utilisation comme antagoniste pan-chimiokine avec un effet antagoniste sur les récepteurs de chimiokine CCR1, CCR2, CCR3, CCR5, CCR9 et CXCR4.

3. Composé selon la revendication 1 ou 2 pour utilisation dans la prévention ou le traitement de métastases cancéreuses formées dans le poumon, le foie, les côtes et les ganglions lymphatiques.

4. Composé selon la revendication 1 ou 2 pour utilisation dans la réduction de la progression de différentes tumeurs malignes, telles que les cancers du sein, gastrique, de la peau (mélanome), de la tête et du cou, du poumon, de l'œsophage, hépatocellulaire, du col de l'utérus, de la thyroïde, des amygdales, colorectal et de la prostate.

5. Composé selon la revendication 1 ou 2 pour utilisation dans la prévention ou le traitement de maladies infectieuses virales associées aux récepteurs de chimiokine telles que VIH-1, VIH-2, SRAS-CoV-2 (COVID-19).

6. Composé selon la revendication 1 ou 2 pour utilisation dans la prévention ou le traitement de maladies liées au système immunitaire telles que le psoriasis, l'arthrite et la dermatite atopique.

7. Composé avec nom IUPAC : 3-chloro-2-{[6-(2-chlorophényl)-2,5-diméthylthiéno[2,3-d]pyrimidin-4-yl]oxy}benzamide (MIC5023) et ses sels pharmaceutiquement acceptables.

8. Composé selon la revendication 7 pour utilisation comme antagoniste pan-chimiokine avec un effet antagoniste sur les récepteurs de chimiokine CCR1, CCR2, CCR3, CCR5, CCR7 et CXCR4.

9. Composé avec nom IUPAC : 3-chloro-2-{[6-(2-chlorophényl)-2-méthylthiéno[2,3-d]pyrimidin-4-yl]oxy}benzamide (MIC5024) et ses sels pharmaceutiquement acceptables.

10. Composé selon la revendication 9 pour utilisation comme antagoniste pan-chimiokine avec un effet antagoniste sur les récepteurs de chimiokine CCR1, CCR2, CCR3, CCR5, CCR7 et CXCR4.
